(19) Europäisches Patentamt
European Patent Office
Office européen des brevets

(11) Publication number: 0 375 164 B1

(12) # EUROPEAN PATENT SPECIFICATION

(45) Date of publication of patent specification: 20.04.94 (51) Int. Cl.[5]: C07H 19/09, A61K 31/70

(21) Application number: 89312146.7

(22) Date of filing: 22.11.89

(54) **Antiviral compounds.**

(30) Priority: 23.11.88 GB 8827339

(43) Date of publication of application:
27.06.90 Bulletin 90/26

(45) Publication of the grant of the patent:
20.04.94 Bulletin 94/16

(84) Designated Contracting States:
AT BE CH DE ES FR GB GR IT LI LU NL SE

(56) References cited:
EP-A- 0 010 205
EP-A- 0 272 065

JOURNAL OF MEDICINAL CHEMISTRY, vol. 26, no. 5, May 1983, Washington, US; E. DE CLERCO: "Nucleic Acid Related Compounds. 40. Synthesis and Biological Activities of 5-Alkynyluracil Nucleosides"

(73) Proprietor: THE WELLCOME FOUNDATION LIMITED
Unicorn House
160 Euston Road
London NW1 2BP(GB)

(72) Inventor: Rahim, Saad George
The Wellcome Research Laboratories
Langley Court Beckenham Kent BR3 3BS(GB)

(74) Representative: Garrett, Michael et al
The Wellcome Research Laboratories
Group Patents and Agreements
Langley Court
Beckenham Kent BR3 3BS (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

## Description

The present invention relates to an esterified 5-propynyl-pyrimidine nucleoside, salts thereof, the use of the nucleoside and salts in medical therapy especially for the treatment or prophylaxis of viral infections, particularly herpes viral infections, and processes for the preparation of the nucleoside and salts.

Within the class of DNA viruses, those of the herpes group represent the cause of the many viral illnesses in man. The group includes herpes simplex virus (HSV), varicella zoster virus (VZV), cytomegalovirus (CMV) and Epstein-Barr virus (EBV).

Varicella zoster virus (VZV) is a herpes virus which causes chicken-pox and shingles. Chicken-pox is the primary form of the disease produced in a host without immunity and in young children is usually a mild illness characterised by a vesicular rash and fever. Shingles or zoster is the recurrent form of the disease which occurs in adults who were previously infected with the virus. The clinical manifestions of shingles are characterised by neuralgia and a vesicular skin rash that is unilateral and dermatomal in distribution. Spread of inflammation may lead to paralysis or convulsions. Coma can occur if the meninges become affected; and disseminated VZV infection can be fatal.

Reappearance of the clinical manifestations of infection by VZV may also be seen in immuno-compromised patients, either as a result of disease or immune modulating therapy for example after an organ transplant. Patients immuno-compromised through disease are now seen in increasing numbers with the advent of Acquired Immune Deficiency Syndrome (AIDS), a condition which severely debilitates the immune system, leaving the patient vulnerable to opportunistic infections.

Infection by cytomegalovirus (CMV) can cause susceptibility to ear and chest infections, failure to thrive or more serious disease states for example microcephaly, hepatosplenomegaly, jaundice or mental retardation. CMV infection can be fatal particularly in immuno-compromised individuals.

Epstein Barr virus (EBV) causes infectious mononucleosis, and is also suggested as a causative agent of nasopharangeal cancer, immunoblastic lymphoma, Burkitt's lymphoma and hairy leukoplakia.

European Patent Publication No. 272065 describes the synthesis and use of 1-(β-D-arabinofuranosyl)-5-alkynyl pyrimidine nucleosides and their pharmaceutically acceptable derivatives in medical therapy particularly in the treatment or prophylaxis of human viral infections such as herpes infections.

We have now discovered that a certain esterified 5-propynyl-substituted pyrimidine nucleoside has advantageous properties on oral administration that renders the nucleoside of particular value in medical therapy especially for the treatment of human viral infections.

The present invention accordingly provides the compound of formula I:

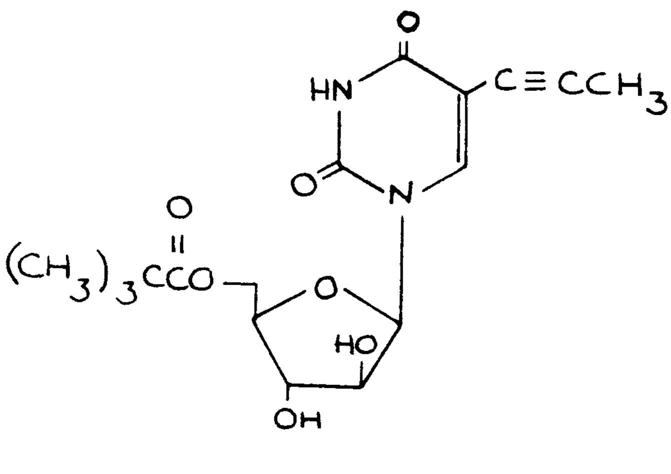

(I)

and pharmaceutically acceptable salts thereof.

The compound of formula I is also characterised by the name 5-prop-1-ynyl-1-(5-0-trimethylacetyl-β-D-arabinofuranosyl)uracil.

Formula I above depicts the compound in its keto form, but it will be appreciated that the compound may also exist in its corresponding enol tautomeric form and hereinafter "a compound of formula I" is used to embrace both the keto and the enol forms, Also hereinafter, the compound of formula I and its pharmaceutically acceptable salts will be referred to as "compounds of the invention".

Tests in rats were carried out on compounds of the invention to determine their bioavailability by measuring the urinary recovery after oral administration, as a percentage of the dose administered. High levels of the parent compound, 1-(β-D-arabinofuranosyl)-5-prop-1-ynyluracil, in the urine indicated good

absorption from the gut. When compared with the parent compound, the compounds of the invention showed a greater than 6-fold increase in absorption.

The improved bioavailability of the compounds of the invention provides a means of achieving high levels of the active parent compound in the plasma, after administration of doses of a compound of the invention which are significantly lower than the doses of the parent compound which would be required to achieve the same high plasma levels. Any side-effects or long term complications which may be associated with repeated high level drug dosing are thereby significantly reduced.

The present invention further provides:-

a) Compounds of the invention for use in medical therapy, for example in the treatment or prophylaxis of a viral infection particularly a herpes viral infection.

b) A method for the treatment of a mammal including a human having a viral infection for example a herpes viral infection which comprises treating said mammal with an antivirally effective amount of a compound of the invention.

c) Use of a compound of the invention in the manufacture of a medicament for the treatment or prophylaxis of a viral infection for example a herpes viral infection.

The compounds of the invention are especially applicable to the treatment or prophylaxis of varicella zoster virus infections and resulting clinical conditions as referred to above. The compounds of the invention are particularly beneficial in the treatment of chicken pox and shingles.

The pharmaceutically acceptable salts of the compound of formula I which may conveniently be used in medical therapy include physiologically acceptable base salts, for example derived from an appropriate base, such as alkali metal (e.g. sodium), alkaline earth metal (e.g. magnesium) and ammonium and $NX_4^+$ (wherein X is $C_{1-4}$ alkyl) salts. Salts having non-physiologically acceptable anions are within the scope of the invention as useful intermediates for the preparation of pharmaceutically acceptable salts and/or for use in non-therapeutic, for example in vitro, studies.

The compounds of the invention may be administered to a mammal including a human ("the recipient") by any route appropriate to the clinical condition to be treated; suitable routes include oral, rectal, nasal, topical (including buccal and sublingual), vaginal and parenteral (including subcutaneous, intramuscular, intravenous, intradermal, intrathecal and epidural). It will be appreciated that the preferred route may vary, for example, according to the age, weight and sex of the recipient and the nature and severity of the condition to be treated.

The amount of the compound of the invention required for the treatment or prophylaxis of a viral infection will depend upon a number of factors including the severity of the condition to be treated and the identity of the recipient and will ultimately be at the discretion of the attendant physician. In general, however, for each of the conditions, a suitable, effective dose will be in the range 1 to 100mg per kilogram body weight per day and most preferably in the range 5 to 30 mg per kilogram body weight per day; an optimum dose is about 15 mg per kilogram body weight per day. The effective dose may be presented as two, three, four or more sub-doses administered at appropriate intervals throughout the day. These sub-doses may be administered in unit dosage forms, for example, containing 10 to 2000 mg, preferably 20 to 500 mg and most preferably 100 to 400 mg of a compound of the invention per unit dosage form. Alternatively, if the condition of the recipient so requires, the dose may be administered as a continuous infusion.

The compounds of the invention may be administered for the treatment or prophylaxis of viral infections alone or in combination with other therapeutic agents, for example, with other antiviral agents such as 9-(2-hydroxy-ethoxymethyl)guanine (acyclovir) used to treat herpes viral infections in particular HSV and most notably those involving the HSV (1) varity, with 3′-deoxy-3′-azidothymidine (zidovudine) or a 2′,3′-dideoxynucleoside for example 2′,3′-dideoxy cytidine, 2′,3′-dideoxyinosine, 2′,3′-dideoxyadenosine or 2′,3′-dideoxy guanosine, used to treat retroviral infections in particular Human Immunodefeciency Virus (HIV) infections, interferons particularly α-interferon and soluble proteins such as CD4, or any other agents such as analagesics or antipyretics which when in combination with a compound of the invention provide a beneficial therapeutic effect.

It is preferable to present the compounds of the invention as pharmaceutical formulations comprising at least one compound of the invention ("the active ingredient"), together with one or more pharmaceutically acceptable carriers therefor and optionally other therapeutic ingredients. The carrier(s) must be "acceptable" in the sense of being compatible with the other ingredients of the formulations and not deleterious to the recipients thereof.

Formulations of the invention include those suitable for administration by any of the aforementioned routes which may conveniently be presented in unit dosage form prepared by any of the methods well known in the art of pharmacy. Such methods include the step of bringing into association the active

ingredient with the carrier which constitutes one or more accessory ingredients. In general the formulations are prepared by uniformly and intimately bringing into association the active ingredient with liquid carriers or finely divided solid carriers or both, and then, if necessary, shaping the product.

Formulations of the invention suitable for oral administration may be presented as discrete units such as capsules, cachets or tablets each containing a predetermined amount of the active ingredient; as a powder or granules; as a solution or a suspension in an aqueous liquid or a non-aqueous liquid; as an edible foam or whip; or as an oil-in-water liquid emulsion or a water-in-oil liquid emulsion. The active ingredient may also be presented as a bolus, or paste or may be contained within liposomes.

A tablet may be made by compression or moulding, optionally with one or more accessory ingredients. Compressed tablets may be prepared by compressing in a suitable machine, the active ingredient in a free-flowing form such as a powder or granules, optionally mixed with a binder (for example povidone, gelatin, hydroxypropylmethyl cellulose), lubricant, inert diluent, disintegrant (for example sodium starch glycollate, cross-linked povidone, cross-linked sodium carboxymethyl cellulose), surface-active or dispersing agent. Moulded tablets may be made by moulding in a suitable machine, a mixture of the powdered active ingredient moistened with an inert liquid diluent. The tablets are optionally coated or scored and may be formulated so as to provide slow or controlled release of the active ingredient therein, using for example, hydroxypropylmethylcellulose in varying proportions to provide the desired release profile, or to be soluble or effervescent when added to liquid.

A capsule may be made by filling a loose or compressed powder on an appropriate filling machine, optionally with one or more additives. Examples of suitable additives include binders such as povidone; gelatin, lubricants, inert diluents and disintegrants as for tablets. Capsules may also be formulated to contain pellets or discrete sub-units to provide slow or controlled release of the active ingredient. This can be achieved by extruding and spheronising a wet mixture of the drug plus an extrusion aid (for example microcrystalline cellulose) plus a diluent such as lactose. The spheroids thus produced can be coated with a semi-permeable membrane (for example ethyl cellulose, Eudragit WE30D) to produce sustained release properties.

An edible foam or whip formulation ideally comprises; 50-70% of an edible oil, particularly a vegetable oil, including corn oil, peanut oil, sunflower oil, olive oil and soybean oil; 2-10% of one or more surfactants particularly lecithin, polyols, polyol polymer esters including glyceryl fatty acid esters, polyglyceryl fatty acid esters (e.g. decaglycerol tetraoleate), or sorbitan fatty acid esters (e.g. sorbitan monostearate); 1-4% of a propellant which is suitable for ingestion, notably a compressed gas propellant especially nitrogen, nitrous oxide or carbon dioxide, or a gaseous hydrocarbon especially propane, butane or isobutane; 0.5-30% of one or more viscosity modifiers of particle size in the range 10-50 microns in diameter, particularly powdered sugars or colloidal silicon dioxide; and optionally 0.5-1% of one or more suitable, non-toxic colourings, flavourings or sweetners. The active ingredient is preferably present in such formulations in a concentration of 10-46%, advantageously 30%. An edible foam or whip formulation as described above may be prepared in a conventional manner, for example by mixing the edible oil, surfactant(s) and any other soluble ingredients, adding the viscosity modifier(s) and milling the mixture to form a uniform dispersion and suspension. The active ingredient is blended into the milled mixture until evenly dispersed. Finally, a metered quantity of propellant is incorporated to the mixture after said mixture has been measured into a suitable dispensing container.

For infections of the eye or other external tissues, for example mouth and skin, the formulations are preferably applied as a topical ointment or cream containing the active ingredient in an amount of, for example, 0.075 to 20% w/w, preferably 0.2 to 15% w/w and most preferably 0.5 to 10% w/w. When formulated in an ointment, the active ingredients may be employed with either a paraffinic or a water-miscible ointment base. Alternatively, the active ingredients may be formulated in a cream with an oil-in-water cream base or as a water-in-oil base.

If desired, the aqueous phase of the cream base may include, for example, at least 40-45% w/w of a polyhydric alcohol, i.e. an alcohol having two or more hydroxyl groups such as propylene glycol, butane-1,3-diol, mannitol, sorbitol, glycerol and polyethylene glycol and mixtures thereof. The topical formulations may include a compound which enhances absorption or penetration of the active ingredient through the skin or other affected areas. Examples of such dermal penetration enhancers include dimethylsulphoxide and related analogues.

The oily phase of an emulsion formulation according to the invention may comprise merely an emulsifier (otherwise known as an emulgent), but desirably comprises a mixture of at least one emulsifier with a fat or an oil or with both a fat and an oil. Preferably, a hydrophilic emulsifier is included together with a lipophilic emulsifier which acts as a stablilizer. It is also preferred to include both an oil and a fat. Together, the emulsifer(s) with or without stabilizer(s) make up the so-called emulsifying wax, and the wax

together with the oil and/or fat make up the so-called emulsifying ointment base which forms the oily phase of the cream formulations.

Emulgents and emulsion stabilizers suitable for use in the formulation of the present invention include Tween 60, Span 80, cetostearyl alcohol, myristyl alcohol, glyceryl mono-stearate and sodium lauryl sulphate.

The choice of suitable oils or fats for the formulation is based on achieving the desired cosmetic properties, since the solubility of the active compound in most oils likely to be used in pharmaceutical emulsion formulations is very low. The cream should preferably be a non-greasy, non-staining and washable product with suitable consistency to avoid leakage from tubes or other containers. Straight or branched chain, mono- or dibasic alkyl esters such as di-isoadipate, isocetyl stearate, propylene glycol diester of coconut fatty acids, isopropyl myristate, decyl oleate, isopropyl palmitate, butyl stearate, 2-ethylhexyl palmitate or a blend of branched chain esters known as Crodamol CAP may be used, the last three being preferred esters. These may be used alone or in combination depending on the properties required. Alternatively, high melting point lipids such as white soft paraffin and/or liquid paraffin or other mineral oils can be used.

Formulations suitable for topical administration to the eye also include eye drops wherein the active ingredient is dissolved or suspended in a suitable carrier, especially an aqueous solvent. The ingredient is preferably present in such formulations in a concentration of 0.5 to 20%, advantageously 0.5 to 10%, particularly about 1.5% w/w.

Formulations suitable for topical administration in the mouth include lozenges comprising the active ingredient in a flavoured material, usually sucrose and acacia or tragacanth; pastilles comprising the active ingredient in an inert material such as gelatin and glycerin, or sucrose and acacia; and mouth-washes comprising the active ingredient in a suitable liquid carrier.

Formulations for rectal administration may be presented as a suppository with a suitable base comprising for example cocoa butter or higher fatty alcohol (e.g. hard wax, European Pharmacopoeia) or triglycerides and saturated fatty acids (e.g. Witepsol).

Formulations suitable for nasal administration wherein the carrier is a solid include a coarse powder having a particle size for example in the range 20 to 500 microns which is administered in the manner in which snuff is taken, i.e. by rapid inhalation through the nasal passage from a container of the powder held close up to the nose. Suitable formulations wherein the carrier is a liquid, for administration as a nasal spray or as nasal drops, include aqueous or oily solutions of the active ingredient.

Formulations suitable for vaginal administration may be presented as pessaries, tampons, creams, gels, pastes, foams or spray formulations containing in addition to the active ingredient such carriers as are known in the art to be appropriate.

Formulations suitable for parenteral administration include aqueous and non- aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example sealed ampoules and vials, and may be stored in a freeze-dried (lyophilized) condition requiring only the addition of the sterile liquid carrier, for example water for injections, immediately prior to use. Extemporaneous injection solutions and suspensions may be prepared from sterile powders, granules and tablets of the kind previously described.

Preferred unit dosage formulations are those containing a daily dose or sub-dose, as herein above recited, or an appropriate fraction thereof, of an active ingredient.

It should be understood that in addition to the ingredients particularly mentioned above, the formulations of this invention may include other agents conventional in the art having regard to the type of formulation in question, for example those suitable for oral administration may include flavouring agents.

The compounds of the invention may be prepared by any method known in the art for the preparation of similar compounds (examples include methods described in UK Patent Specification No. 1 601 020, EP Publications Nos. 61283 and 27065, or Robins M.J. and Barr, P.J., J.Org. Chem. (1983) 48, 1854-1862), as well as the processes described in the Examples given hereinafter.

The present invention also provides processes for the preparation of a compound of the invention comprising:-

A. reacting a compound of formula II

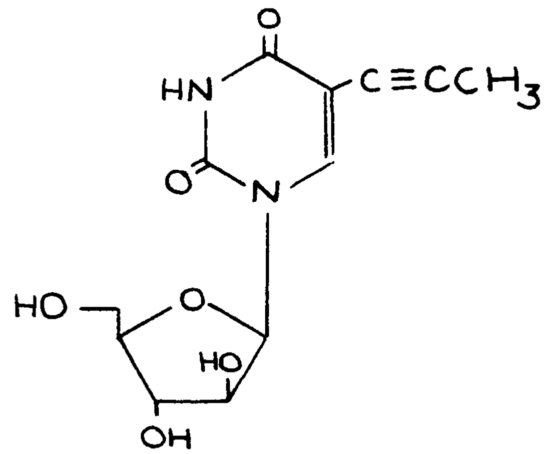

(II)

with a compound serving to provide the trimethylacetyl group at the 5-position of the sugar moiety; or

B. reacting a compound of formula III

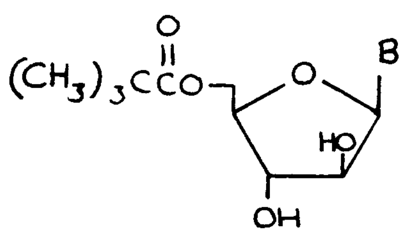

(III)

wherein B is a purine or pyrimidine base (other than 5-prop-1-ynyl-uracil),with 5-prop-1-ynyluracil; and optionally thereafter or simultaneously therewith, where the resulting compound is a compound of formula I, converting it into a salt thereof; or where the resulting compound is a salt, converting it into a different salt or a compound of formula I.

With regard to process A, a compound of formula I may be prepared from a compound of formula II, the preparation of which is described in EP Publication No. 272065, for example by acylation using an appropriate acylating agent such as trimethylacetylhalide (e.g. chloride) or trimethylacetic anhydride, advantageously in the presence of a base such as pyridine or triethylamine which may also serve as a solvent medium for the reaction at a temperature in the range of 0°-70°C advantageously 0°-30°C, preferably 0-15°C. The ratio of acylating agent to compound of formula II is preferably about 1.2:1w/w.

Again, a compound of formula I may be prepared from a compound of formula II by transesterification using an appropriate ester of trimethylacetic acid (e.g. the methyl ester) in the presence of a base such as pyridine or triethylamine which may also serve as a solvent medium for the reaction.

In addition, the esterification reaction may be carried out for example in a solvent such as pyridine or dimethylformamide in the presence of a coupling agent such as N,N′-dicyclohexylcarbodiimide, optionally in the presence of a catalytic base such as 4-dimethylaminopyridine using the acylating agents referred to above. Subsequently, the ester obtained as reaction product may be isolated in conventional manner.

With regard to process B, group B is preferably a purine or pyrimidine base capable of donating the esterified sugar to a 5-prop-1-ynyluracil base using for example an enzyme such as a phosphorylase enzyme in the presence of a phosphate salt at a pH of 5.0 - 9.0 and a temperature of 15° - 90°C, advantageously 40° - 60°C.

Salts according to the invention may also be prepared in conventional manner for example by reaction of a compound of formula II with an appropriate base to form the corresponding base salt followed by acylation. Again, salts may be prepared by reaction of the esterified compound with a base such as sodium hydride to form the corresponding sodium salt. Other derivatives according to the invention can also be prepared in conventional manner.

The following examples are for illustration of the present invention and should not be considered as limiting in any way:-

Example 1

5-Prop-1-ynyl-1-(5-0-trimethylacetyl-β-D-arabinofuranosyl)uracil

To a stirred solution of 1-(β-D-arabinofuranosyl)-5-prop-1-ynyluracil (0.28g, 1mmol, synthesised by the method described in EP Publication No. 272065) in dry pyridine (5ml) at 0 °C under dry nitrogen, was added dropwise a solution of trimethylacetylchloride (0.15ml, 0.14g, 1.2mmol) in dry dichloromethane (5ml) over a period of 10 minutes. The mixture was stirred at 0 °C for 90 minutes then at room temperature for 2 hours. The solvent was evaporated under reduced pressure, and residual pyridine co-evaporated with portions of ethanol (3 x 25ml) to give an oil. Chromatographic separation on a silica gel column eluting with 8% methanol/dichloromethane gave pure product which was triturated with ether to give a white solid identified as the title compound.

Mpt: 204-210 °C.

| Analysis | Calc :<br>Found : | C-55.74,<br>C-55.95, | H-6.011,<br>H-6.006, | N-7.65%<br>N-7.525%. |
|---|---|---|---|---|

δ($d_6$ DMSO) 11.55(1H,6s,NH), 7.58(1H,s,H-6), 6.0(1H,d,H-1′), 5.72(1H,d,OH-2′), 5.62(1H,m,OH-3′), 4.4-4.13-(2H,m,H-5′), 4.08-3.89 (3H,m,H-2′,H-3′,H-4′), 1.97(3H,s,C≡$CCH_3$), 1.19ppm(9H,s,tBu).

Example 2

Sodium salt of 5-Prop-1-ynyl-1-(5-trimethylacetyl-β-D-arabinofuranosyl)uracil

A suspension of sodium hydride (0.05g of 80% w/v suspension in oil, 1.66mmol, washed several times with dry tetrahydrofuran) in dry tetrahydrofuran (4ml) was added to a stirred solution of 5-prop-1-ynyl-1-(5-trimethylacetyl-β-D-arabinofuranosyl)uracil (0.06g, 1.64mmol) in dry tetrahydrofuran, ensuring complete exclusion of moisture. The solvent was evaporated after 1 hour to give 0.1g of the required sodium salt.

Example A

| | Opthalmic Solution |
|---|---|
| Active ingredient | 0.5 g |
| Sodium chloride, analytical grade | 0.9 g |
| Thiomersal | 0.001 g |
| Purified water to | 100 ml |
| pH adjusted to | 7.5 |

Example B: Tablet Formulations

The following formulations A, B and C are prepared by wet granulation of the ingredients with a solution of povidone, followed by addition of magnesium stearate and compression.

Formulation A

| | mg/tablet | mg/tablet |
|---|---|---|
| (a) Active ingredient | 250 | 250 |
| (b) Lactose B.P. | 210 | 26 |
| (c) Povidone B.P. | 15 | 9 |
| (d) Sodium Starch Glycollate | 20 | 12 |
| (e) Magnesium Stearate | 5 | 3 |
| | 500 | 300 |

Formulation B

| | mg/tablet | mg/tablet |
|---|---|---|
| (a) Active ingredient | 250 | 250 |
| (b) Lactose | 150 | - |
| (c) Avicel PH 101 | 60 | 26 |
| (d) Povidone B.P. | 15 | 9 |
| (e) Sodium Starch Glycollate | 20 | 12 |
| (f) Magnesium Stearate | 5 | 3 |
| | 500 | 300 |

Formulation C

| | mg/tablet |
|---|---|
| Active ingredient | 100 |
| Lactose | 200 |
| Starch | 50 |
| Povidone | 5 |
| Magnesium stearate | 4 |
| | 359 |

The following formulations, D and E, are prepared by direct compression of the admixed ingredients. The lactose used in formulation E is of the direct compression type.

Formulation D

| | mg/tablet |
|---|---|
| Active Ingredient | 250 |
| Pregelatinised Starch NF15 | 150 |
| | 400 |

Formulation E

| | mg/tablet |
|---|---|
| Active Ingredient | 250 |
| Lactose | 150 |
| Avicel | 100 |
| | 500 |

Formulation F (Controlled Release Formulation)

The formulation is prepared by wet granulation of the ingredients (below) with a solution of povidone followed by the addition of magnesium stearate and compression.

| | mg/tablet |
|---|---|
| (a) Active Ingredient | 500 |
| (b) Hydroxypropylmethylcellulose (Methocel K4M Premium) | 112 |
| (c) Lactose B.P. | 53 |
| (d) Povidone B.P.C. | 28 |
| (e) Magnesium Stearate | 7 |
| | 700 |

Drug release takes place over a period of about 6-8 hours and was complete after 12 hours.

Example C: Capsule Formulations

Formulation A

A capsule formulation is prepared by admixing the uncompressed ingredients of Formulation D in Example B above and filling into a two-part hard gelatin capsule. Formulation B (infra) is prepared in a similar manner.

Formulation B

| | mg/capsule |
|---|---|
| (a) Active ingredient | 250 |
| (b) Lactose B.P. | 143 |
| (c) Sodium Starch Glycollate | 25 |
| (d) Magnesium Stearate | 2 |
| | 420 |

Formulation C

| (a) Active ingredient | mg/capsule 250 |
|---|---|
| (b) Macrogol 4000 BP | 350 |
| | 600 |

Capsules are prepared by melting the Macrogol 4000 BP, dispersing the active ingredient in the melt and filling the melt into a two-part hard gelatin capsule.

Formulation D

| | mg/capsule |
|---|---|
| Active ingredient | 250 |
| Lecithin | 100 |
| Arachis Oil | 100 |
| | 450 |

Capsules are prepared by dispersing the active ingredient in the lecithin and arachis oil and filling the dispersion into soft, elastic gelatin capsules.

Formulation E (Controlled Release Capsule)

The following controlled release capsule formulation is prepared by extruding ingredients (a), (b), and (c) below, using an extruder, followed by spheronisation of the extrudate and drying. The dried pellets are then coated with release- controlling membrane (d) and filled into a two-piece, hard gelatin capsule.

| | mg/capsule |
|---|---|
| (a) Active Ingredient | 250 |
| (b) Microcrystalline Cellulose | 125 |
| (c) Lactose BP | 125 |
| (d) Ethyl Cellulose | 13 |
| | 513 |

Injectable Formulation

Example D:

| | |
|---|---|
| Active ingredient | 0.200 g |
| Sterile, pyrogen free phosphate buffer (pH 7.0) to | 10 ml |

The active ingredient is dissolved in most of the phosphate buffer (35- 40 ° C), then made up to volume and filtered through a sterile micropore filter into a sterile 10ml amber glass vial (type 1) and sealed with sterile closures and overseals.

Example E : Intramuscular Injection

| | |
|---|---|
| Active Ingredient | 0.20 g |
| Benzyl Alcohol | 0.10 g |
| Glycofurol 75 | 1.45 g |
| Water for Injection | q.s. to 3.00 ml |

The active ingredient is dissolved in the glycofurol. The benzyl alcohol is then added and dissolved, and water added to 3 ml. The mixture is then filtered through a sterile micropore filter and sealed in sterile 3 ml glass vials (type 1).

Example F : Syrup Suspension

| | | |
|---|---|---|
| Active ingredient | | 0.2500 g |
| Sorbitol Solution | | 1.5000 g |
| Glycerol | | 2.0000 g |
| Dispersible Cellulose | | 0.0750 g |
| Sodium Benzoate | | 0.0050 g |
| Flavour, Peach 17.42.3169 | | 0.0125 ml |
| Purified Water | q.s. to | 5.0000 ml |

The sodium benzoate is dissolved in a portion of the purified water and the sorbitol solution added. The active ingredient is added and dispersed. In the glycerol is dispersed the thickener (dispersible cellulose). The two dispersions are mixed and made up to the required volume with the purified water.

Example H : Suppository

| | mg/suppository |
|---|---|
| Active Ingredient (63µm) | 250 |
| Hard Fat, BP (Witepsol H15 - Dynamit NoBel) | 1770 |
| | 2020 |

* The active ingredient is used as a powder wherein at least 90% of the particles are of 63µm diameter or less.

One-fifth of the Witepsol H15 is melted in a steam-jacketed pan at 45°C maximum. The active ingredient is sifted through a 200µm sieve and added to the molten base with mixing, using a silverson fitted with a cutting head, until a smooth dispersion is achieved. Maintaining the mixture at 45°C, the remaining Witepsol H15 is added to the suspension and stirred to ensure a homogenous mix. The entire suspension is passed through a 250µm stainless steel screen and, with continuous stirring, is allowed to cool to 40°C. At a temperature of 38°C to 40°C 2.02g of the mixture is filled into suitable plastic moulds. The suppositories are allowed to cool to room temperature.

Example H : Pessaries

| | mg/pessary |
|---|---|
| Active ingredient 63µm | 250 |
| Anhydrous Dextrose | 380 |
| Potato Starch | 363 |
| Magnesium Stearate | 7 |
| | 1000 |

The above ingredients are mixed directly and pessaries prepared by direct compression of the resulting mixture.

Example I : Topical formulation

| | Cream |
|---|---|
| Active compound | 5.00g |
| Glycerol | 2.00g |
| Cetostearyl alcohol | 6.75g |
| Sodium lauryl sulphate | 0.75g |
| White soft paraffin | 12.50g |
| Liquid paraffin | 5.00g |
| Chlorocresol | 0.10g |
| Purified water to | 100.00g |

The active compound is dissolved in a mixture of purified water and glycerol and heated to 70°C. The remaining ingredients are heated together at 70°C. The two parts are added together and emulsified. Cooled and filled into containers.

Determination of Oral Bioavailability

Long Evans Rats were administered the compound of Example 1 or the parent compound by gavage at a dose of 50 mg/kg. The urine was collected for 24 and 48 hours post-dose, ultrafiltered, and analysed by reverse-phase high-pressure liquid chromatography. The oral bioavailability of the Example 1 compound and the parent compound was expressed as the percent of the dose excreted in the urine over the 48 hour period of collection, as 1-(β-D-arabinofuranosyl)-5-prop-1-ynyluracil, i.e. the parent compound.

| Compound | Urinary Recovery (% of dose) |
|---|---|
| Example 1 | 64.89 |
| Parent Compound | 9.70 |

Toxicity

Cell toxicity is assessed in a cell growth inhibition assay. Subconfluent cultures of Vero cells grown on 96-well microtiter dishes are exposed to different dilutions of drug, and cell viability determined daily on replicate cultures using uptake of a tetrazolium dye (MTT). The concentration required for a 50% inhibition of cell vaibility at 96 hours is termed $CCID_{50}$.

| Example | $CCID_{50}$ (μM) at 96 hr |
|---|---|
| 1 | 477 |

**Claims**
**Claims for the following Contracting States : AT, BE, CH, DE, FR, GB, IT, LI, LU, NL, SE**

1. A compound of formula I:

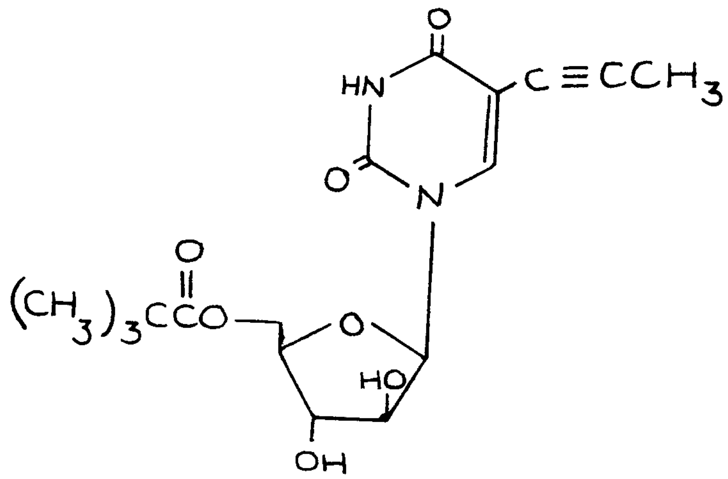

(I)

or a salt thereof.

2. 5-Prop-1-ynyl-1(5-O-trimethylacetyl-β-D-arabinofuranosyl)uracil.

3. A compound of formula I according to claim 1 or a pharmaceutically acceptable salt thereof, for use in medical therapy.

4. A compound of formula I according to claim 1 or a pharmaceutically acceptable salt thereof, for use in the treatment or prophylaxis of a viral infection.

5. A compound of formula I according to claim 1 or a pharmaceutically acceptable salt thereof, for use in the treatment or prophylaxis of a herpes viral infection.

6. A compound of formula I according to claim 1 or a pharmaceutically acceptable salt thereof, for use in the treatment or prophylaxis of a varicella zoster virus infection.

7. A compound of formula I according to claim 1 or a pharmaceutically acceptable salt thereof, for use in the treatment or prophylaxis of chicken-pox.

8. A compound of formula I according to claim 1 or a pharmaceutically acceptable salt thereof, for use in the treatment or prophylaxis of shingles.

9. Use of a compound of formula I according to claim 1 or a pharmaceutically acceptable salt thereof, in the manufacture of a medicament for the treatment or prophylaxis of a viral infection.

**10.** A process for the preparation of a compound of formula I:

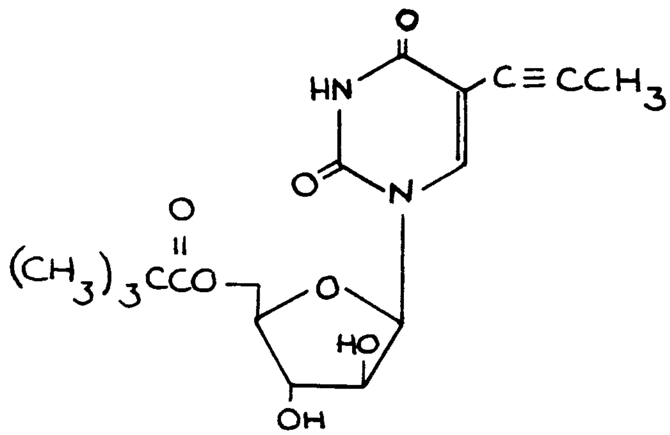

(I)

or a salt thereof, comprising:

A. reacting a compound of formula II

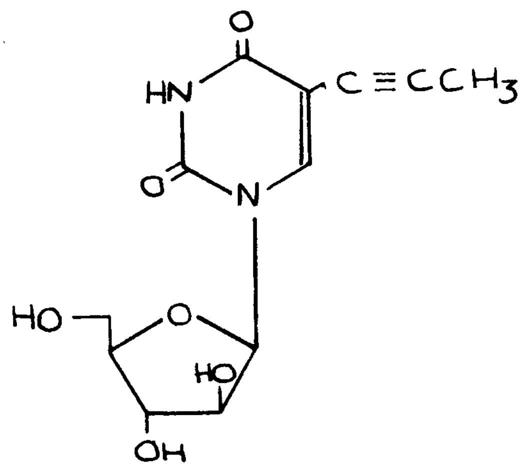

(II)

with a compound serving to provide the trimethylacetyl group at the 5-position of the sugar moiety; or

B. reacting a compound of formula III

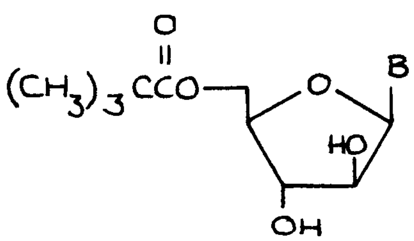

(III)

wherein B is a purine or pyrimidine base, (other than 5-prop-1-ynyluracil) with 5-prop-1-ynyluracil; and optionally thereafter or simultaneously therewith, where the resulting compound is a compound of formula I, converting it into a salt thereof or, where the resulting compound is a salt, converting it into a different salt or a compound of formula I.

**11.** A pharmaceutical formulation comprising a compound of formula I :

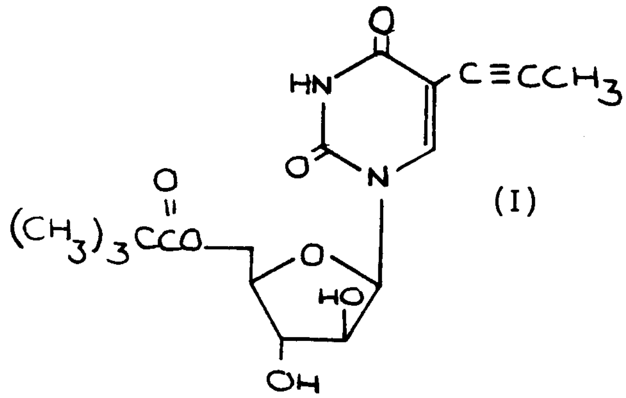

(I) (I)

or a pharmaceutically acceptable salt thereof and one or more pharmaceutically acceptable carriers.

**12.** A pharmaceutical formulation according to claim 11 suitable for oral administration.

**13.** A pharmaceutical formulation according to claim 12 in the form of a tablet.

**14.** A pharmaceutical formulation according to claim 12 in the form of a liquid.

**Claims for the following Contracting States: ES, GR**

**1.** A process for the preparation of a compound of formula I:

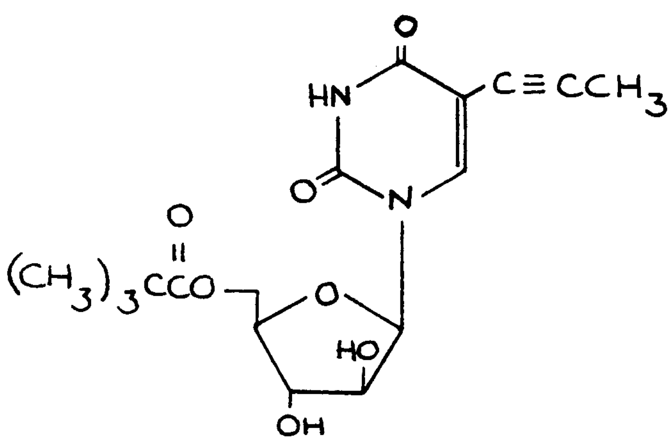

(I)

or a salt thereof, comprising:

A. reacting a compound of formula II

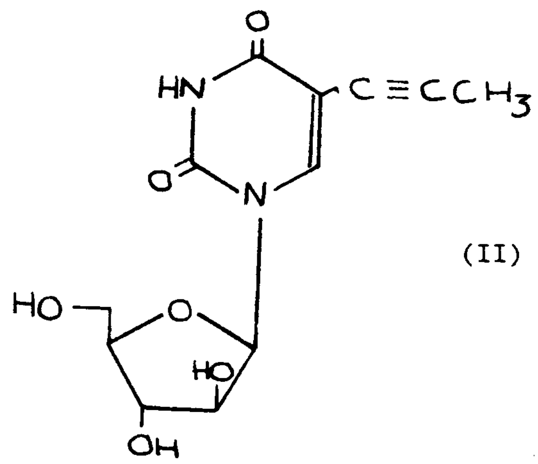

(II)

with a compound serving to provide the trimethylacetyl group at the 5-position of the sugar moiety; or

B. reacting a compound of formula III

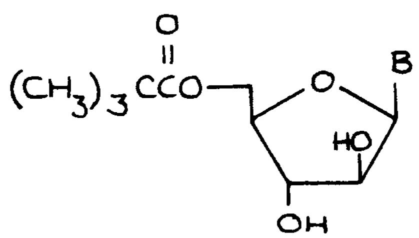

(III)

wherein B is a purine or pyrimidine base, (other than 5-prop-1-ynyluracil) with 5-prop-1-ynyluracil; and optionally thereafter or simultaneously therewith, where the resulting compound is a compound of formula I, converting it into a salt thereof or, where the resulting compound is a salt, converting it into a different salt or a compound of formula I.

**2.** A process according to claim 1 for the preparation of 5-prop-1-ynyl-1-(5-0-trimethylacetyl-$\beta$-D-arabinofuranosyl)uracil.

**3.** A process for the preparation of a pharmaceutical formulation containing a compound of formula I :

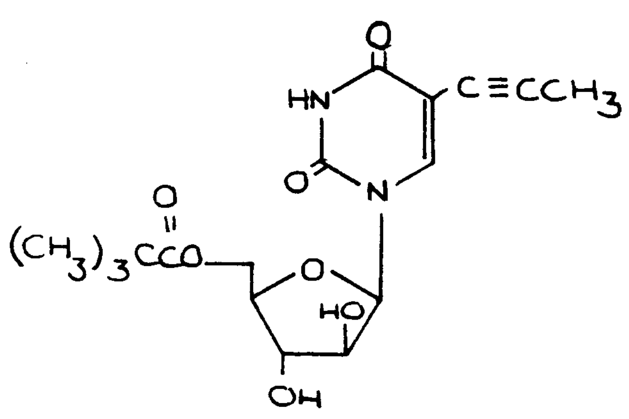

(I)

or a pharmaceutically acceptable salt thereof which comprises preparing a compound of formula I, its

enol form, or a pharmaceutically acceptable salt thereof by :

A. reacting a compound of formula II

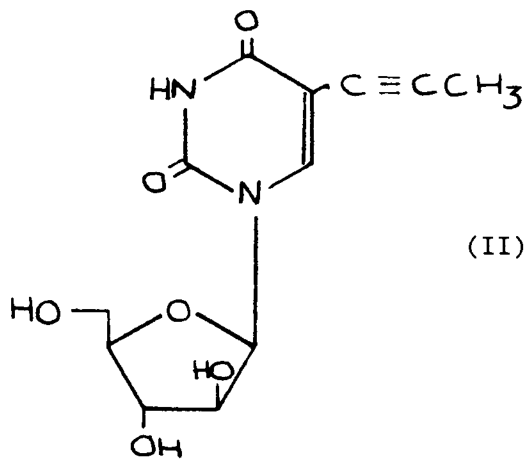

(II)

with a compound serving to provide the trimethylacetyl group at the 5-position of the sugar moiety; or

B. reacting a compound of formula III

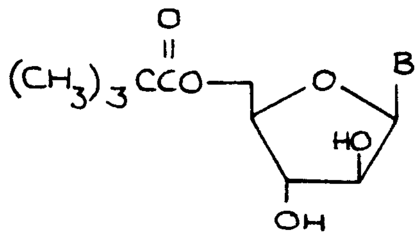

(III)

wherein B is a purine or pyrimidine base, (other than 5-prop-1-ynyluracil) with 5-prop-1-ynyluracil; and optionally thereafter or simultaneously therewith, where the resulting compound is a compound of formula I, converting it into a pharmaceutically acceptable salt thereof or, where the resulting compound is a salt, converting it into a different pharmaceutically acceptable salt or a compound of formula I; and thereafter bringing the resulting compound into association with a pharmaceutically acceptable carrier therefor.

**Patentansprüche**

**Patentansprüche für folgende Vertragsstaaten : AT, BE, CH, DE, FR, GB, IT, LI, LU, NL, SE**

1. Verbindung der Formel I

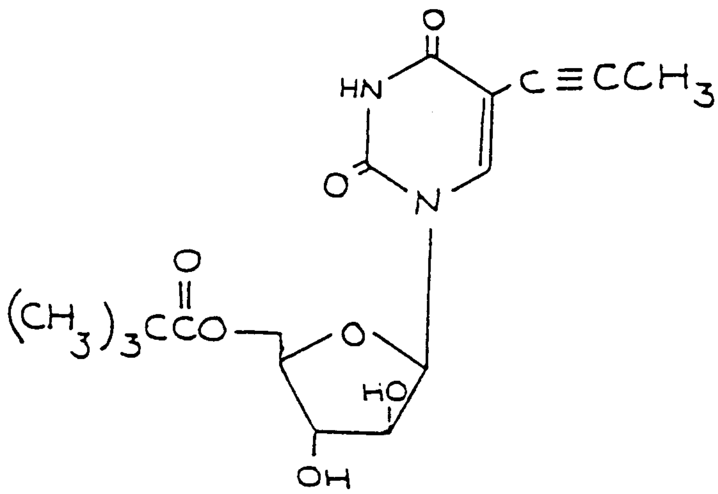

(I)

oder eines seiner Salze.

2. 5-Prop-1-inyl-1-(5-O-trimethylacetyl-β-D-arabinofuranosyl)uracil.

3. Verbindung der Formel I gemäß Anspruch 1 oder eines ihrer pharmazeutisch annehmbaren Salze zur Verwendung in der medizinischen Therapie.

4. Verbindung der Formel I gemäß Anspruch 1 oder eines ihrer pharmazeutisch annehmbaren Salze zur Verwendung bei der Behandlung oder Prophylaxe einer Virusinfektion.

5. Verbindung der Formel I gemäß Anspruch 1 oder eines ihrer pharmazeutisch annehmbaren Salze zur Verwendung bei der Behandlung oder Prophylaxe einer Herpes-Virusinfektion.

6. Verbindung der Formel I gemäß Anspruch 1 oder eines ihrer pharmazeutisch annehmbaren Salze zur Verwendung bei der Behandlung oder Prophylaxe einer Varicella-Zoster-Virusinfektion.

7. Verbindung der Formel I gemäß Anspruch 1 oder eines ihrer pharmazeutisch annehmbaren Salze zur Verwendung bei der Behandlung oder Prophylaxe von Windpocken.

8. Verbindung der Formel I gemäß Anspruch 1 oder eines ihrer pharmazeutisch annehmbaren Salze zur Verwendung bei der Behandlung oder Prophylaxe von Gürtelrose.

9. Verwendung einer Verbindung der Formel I gemäß Anspruch 1 oder eines ihrer pharmazeutisch annehmbaren Salze bei der Herstellung eines Medikaments zur Behandlung oder Prophylaxe einer viralen Infektion.

**10.** Verfahren zur Herstellung einer Verbindung der Formel I

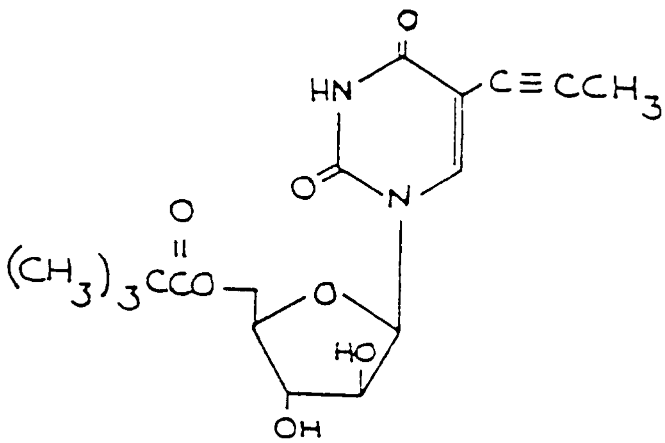

(I)

oder eines ihrer Salze, umfassend:

A. Umsetzung einer Verbindung der Formel II

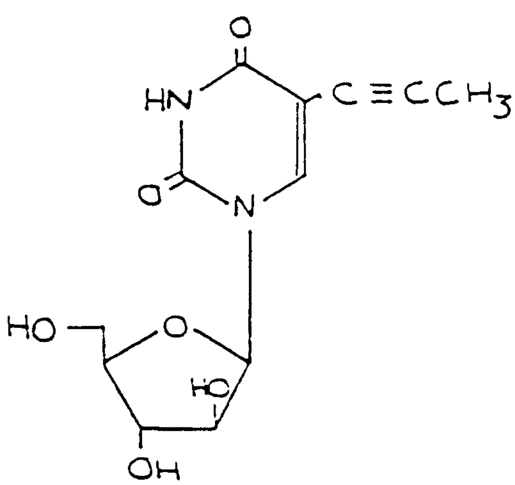

(II)

mit einer Verbindung, die dazu dient, die Trimethylacetylgruppe an der 5-Position der Zuckereinheit zur Verfügung zu stellen; oder

B. Umsetzung einer Verbindung der Formel III

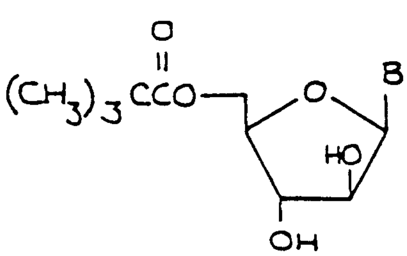

(III)

worin B eine Purin- oder Pyrimidinbase ist (verschieden von 5-Prop-1-inyluracil) mit 5-Prop-1-inyluracil; und wahlweise danach oder gleichzeitig, wenn die resultierende Verbindung eine Verbindung der Formel I ist, Umwandlung in eines ihrer Salze, oder, wenn die resultierende Verbindung ein Salz ist, Umwandlung in ein anderes Salz oder eine Verbindung der Formel I.

**11.** Pharmazeutische Formulierung, umfassend eine Verbindung der Formel I:

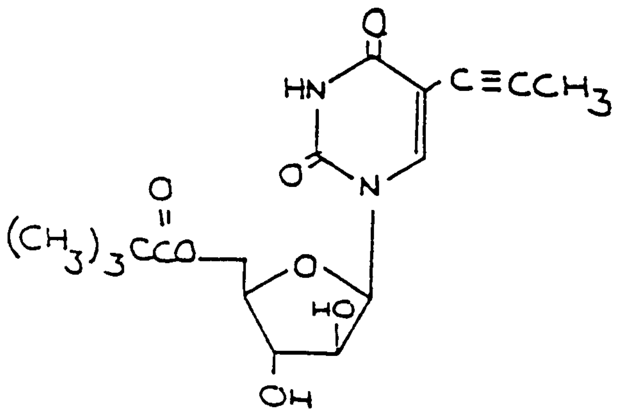

(I)

oder eines ihrer pharmazeutisch annehmbaren Salze und einen oder mehrere pharmazeutisch annehmbare Träger.

**12.** Pharmazeutische Formulierung gemäß Anspruch 11, geeignet zur oralen Verabreichung.

**13.** Pharmazeutische Formulierung gemäß Anspruch 12 in Form einer Tablette.

**14.** Pharmazeutische Formulierung gemäß Anspruch 12 in Form einer Flüssigkeit.

**Patentansprüche für folgende Vertragsstaaten : ES, GR**

**1.** Verfahren zur Herstellung einer Verbindung der Formel I

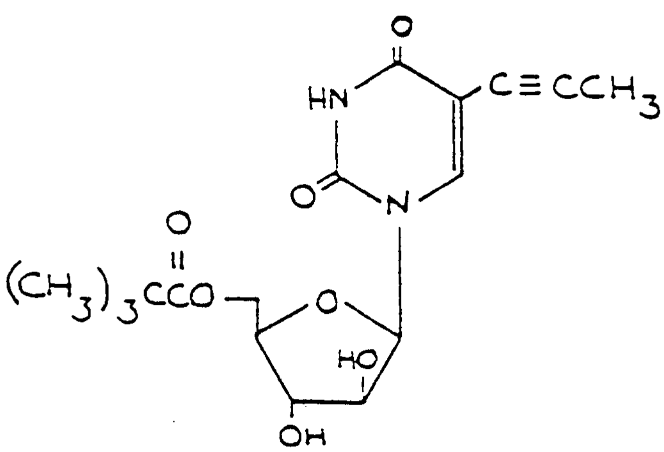

(I)

oder eines ihrer Salze,
umfassend:

A. Umsetzung einer Verbindung der Formel II

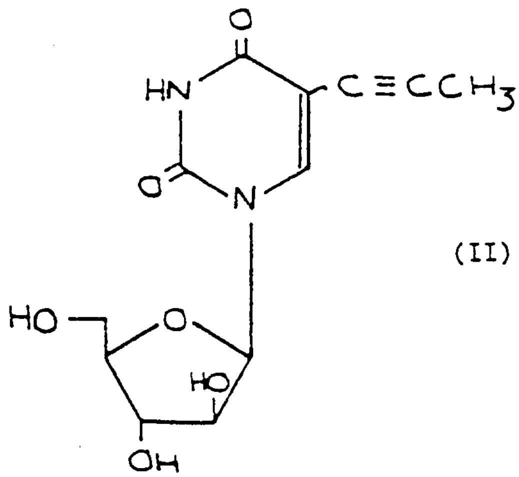

(II)

mit einer Verbindung, die dazu dient, die Trimethylacetylgruppe an der 5-Position der Zuckereinheit zur Verfügung zu stellen; oder

B. Umsetzung einer Verbindung der Formel III

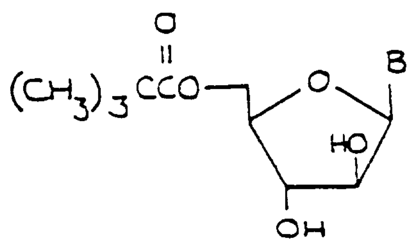

(III)

worin B eine Purin- oder Pyrimidinbase ist (verschieden von 5-Prop-1-inyluracil) mit 5-Prop-1-inyluracil; und wahlweise danach oder gleichzeitig, wenn die resultierende Verbindung eine Verbindung der Formel I ist, Umwandlung in eines ihrer Salze, oder, wenn die resultierende Verbindung ein Salz ist, Umwandlung in ein anderes Salz oder eine Verbindung der Formel I.

**2.** Verfahren gemäß Anspruch 1 zur Herstellung von 5-Prop-1-inyl-1-(5-O-trimethylacetyl-$\beta$-D-arabinofuranoxyl)uracil.

**3.** Verfahren zur Herstellung einer pharmazeutischen Formulierung, enthaltend eine Verbindung der Formel I

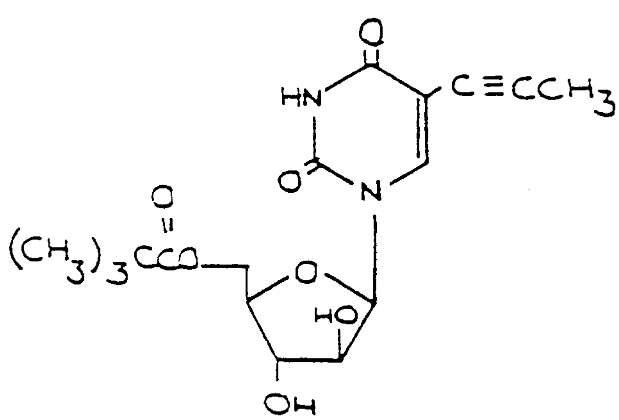

(I)

oder eines ihrer pharmazeutisch annehmbaren Salze, umfassend die Herstellung einer Verbindung der Formel I, ihrer Enolform oder eines ihrer pharmazeutisch annehmbaren Salze durch:

A. Umsetzung einer Verbindung der Formel II

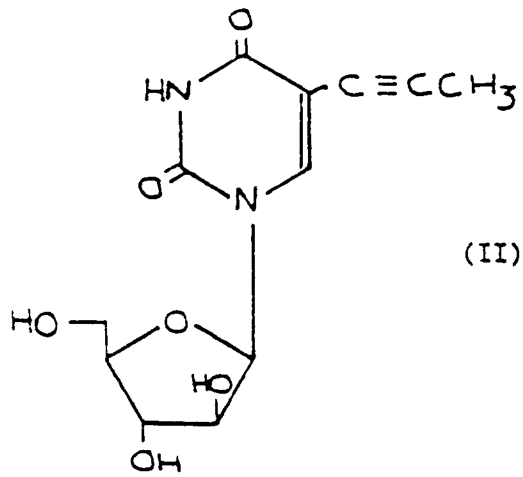

(II)

mit einer Verbindung, die dazu dient, die Trimethylacetylgruppe an der 5-Position der Zuckereinheit zur Verfügung zu stellen; oder

B. Umsetzung einer Verbindung der Formel III

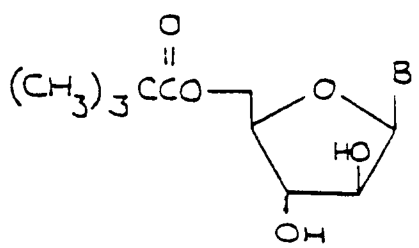

(III)

worin B eine Purin- oder Pyrimidinbase ist (verschieden von 5-Prop-1-inyluracil) mit 5-Prop-1-inyluracil; und wahlweise danach oder gleichzeitig, wenn die resultierende Verbindung eine Verbindung der Formel I ist, Umwandlung in eines ihrer pharmazeutisch annehmbaren Salze, oder, wenn die resultierende Verbindung ein Salz ist, Umwandlung in ein anderes pharmazeutisch annehmbares Salz oder eine Verbindung mit der Formel I; und danach In-Verbindung-Bringen der resultierenden Verbindung mit einem pharmazeutisch annehmbaren Träger dafür.

**Revendications**

**Revendications pour les Etats contractants suivants : AT, BE, CH, DE, FR, GB, IT, LI, LU, NL, SE**

1. Composé de formule I :

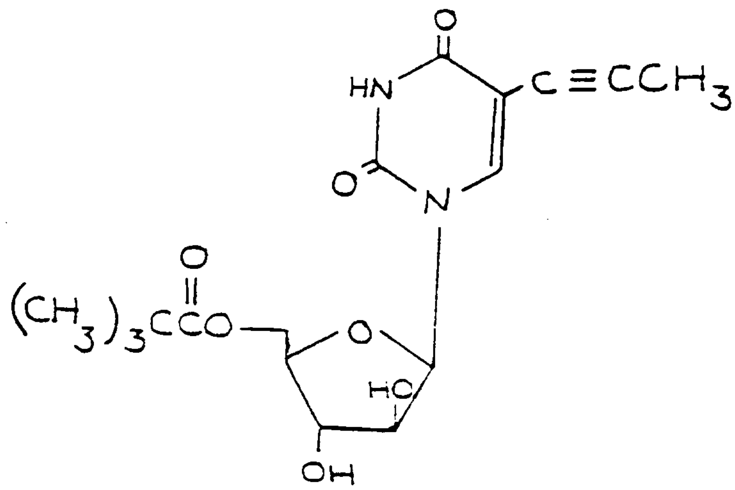

(I)

ou un sel de celui-ci.

2. 5-Prop-1-ynyl-1-(5-O-triméthylacétyl-$\beta$-D-arabinofuranosyl)uracile.

3. Composé de formule I suivant la revendication 1, ou un sel pharmaceutiquement acceptable de celui-ci, à utiliser en thérapie médicale.

4. Composé de formule I suivant la revendication 1, ou un sel pharmaceutiquement acceptable de celui-ci, à utiliser dans le traitement ou la prophylaxie d'une infection virale.

5. Composé de formule I suivant la revendication 1, ou un sel pharmaceutiquement acceptable de celui-ci, à utiliser dans le traitement ou la prophylaxie d'une infection par le virus de l'herpès.

6. Composé de formule I suivant la revendication 1, ou un sel pharmaceutiquement acceptable de celui-ci, à utiliser dans le traitement ou la prophylaxie d'une infection par le virus de la varicelle.

7. Composé de formule I suivant la revendication 1, ou un sel pharmaceutiquement acceptable de celui-ci, à utiliser dans le traitement ou la prophylaxie de la varicelle.

8. Composé de formule I suivant la revendication 1, ou un sel pharmaceutiquement acceptable de celui-ci, à utiliser dans le traitement ou la prophylaxie du zona.

9. Utilisation d'un composé de formule I suivant la revendication 1, ou d'un sel pharmaceutiquement acceptable de celui-ci, dans la fabrication d'un médicament pour le traitement ou la prophylaxie d'une infection virale.

**10.** Procédé de préparation d'un composé de formule I :

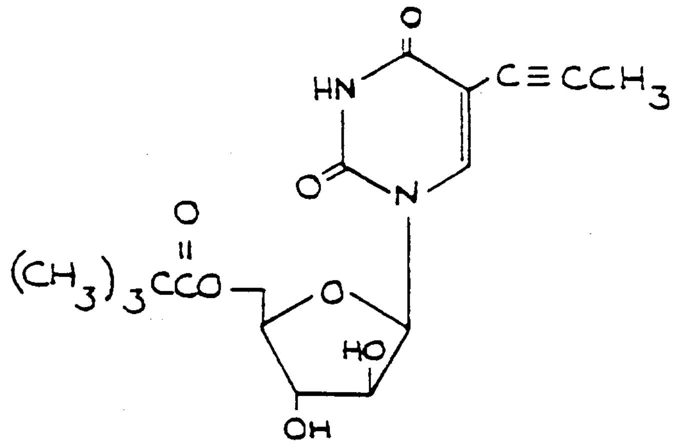

(I)

ou d'un sel de celui-ci, comprenant :

A. la réaction d'un composé de formule II :

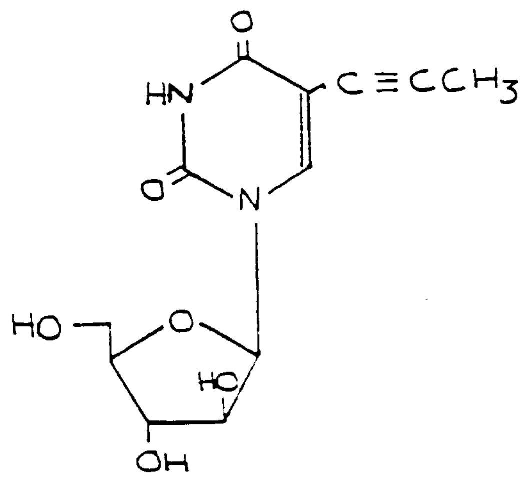

(II)

avec un composé servant à fournir le radical triméthylacétyle en position 5 du résidu sucre, ou

B. la réaction d'un composé de formule III :

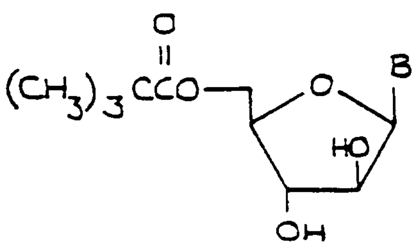

(III)

où B est une base purique ou pyrimidinique (autre que 5-prop-1-ynyluracile) avec du 5-prop-1-ynyluracile et, facultativement, après ou simultanément, quand le composé résultant est un composé de formule I, conversion en un sel de celui-ci ou, quand le composé résultant est un sel, conversion en un sel différent ou en un composé de formule I.

**11.** Formulation pharmaceutique comprenant un composé de formule I :

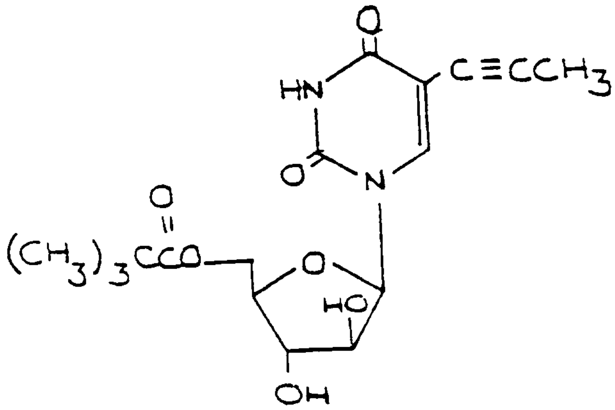

(I)

ou un sel pharmaceutiquement acceptable de celui-ci et un ou plusieurs excipients pharmaceutiquement acceptables.

**12.** Formulation pharmaceutique suivant la revendication 11, appropriée pour une administration orale.

**13.** Formulation pharmaceutique suivant la revendication 12, sous la forme de comprimés.

**14.** Formulation pharmaceutique suivant la revendication 12, sous la forme d'un liquide.

**Revendications pour les Etats contractants suivants : ES, GR**

**1.** Procédé de préparation d'un composé de formule I :

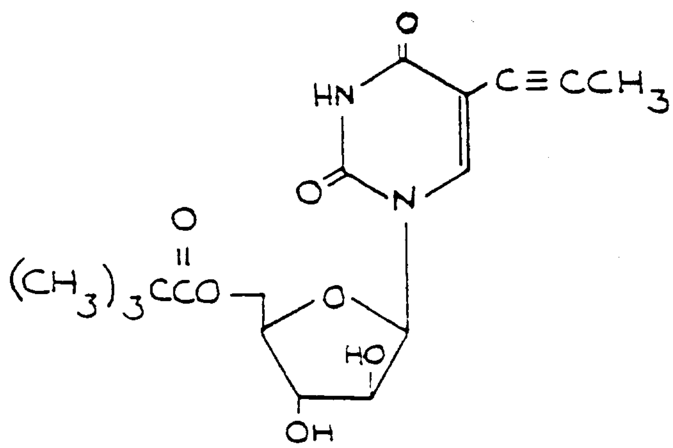

(I)

ou un sel de celui-ci, comprenant :

A. la réaction d'un composé de formule II :

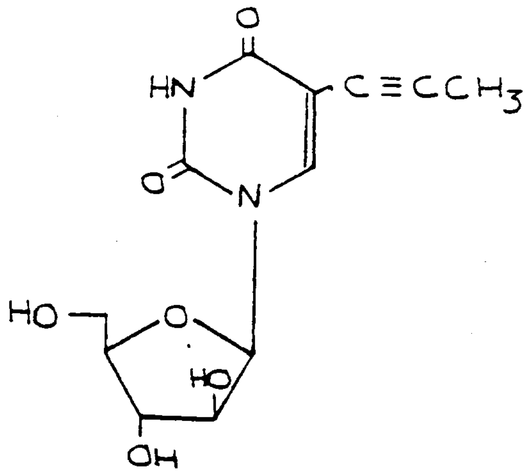

(II)

avec un composé servant à fournir le radical triméthylacétyle en position 5 du résidu sucre, ou

B. la réaction d'un composé de formule III :

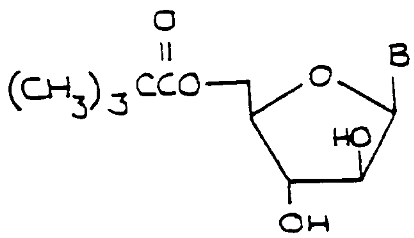

(III)

où B est une base purique ou pyrimidinique (autre que 5-prop-1-ynyluracile) avec du 5-prop-1-ynyluracile et, facultativement, après ou simultanément, quand le composé résultant est un composé de formule I, conversion en un sel de celui-ci ou, quand le composé résultant est un sel, conversion en un sel différent ou en un composé de formule I.

**2.** Procédé suivant la revendication 1, pour la préparation du 5-prop-1-ynyl-1-(5-O-triméthylacétyl-$\beta$-D-arabinofuranosyl)uracile.

**3.** Procédé de préparation d'une formulation pharmaceutique contenant un composé de formule I :

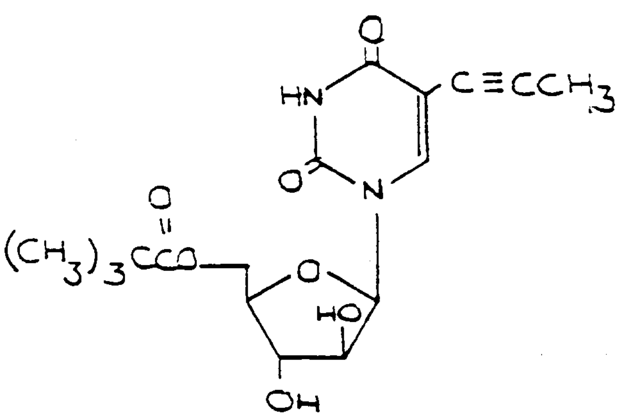

(I)

ou un sel pharmaceutiquement acceptable de celui-ci, qui comprend la préparation d'un composé de

formule I, sa forme énolique ou un sel pharmaceutiquement acceptable de ceux-ci, par :

A. la réaction d'un composé de formule II :

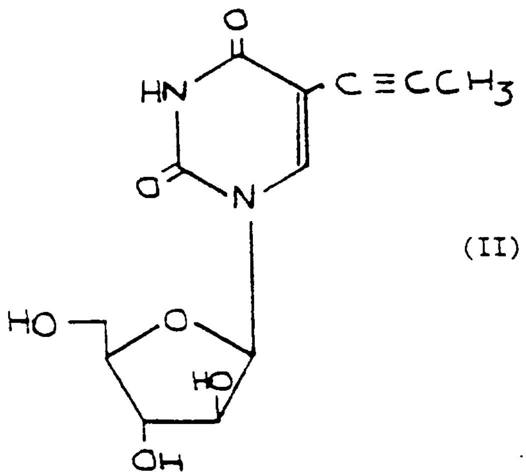

(II)

(II)

avec un composé servant à fournir le radical triméthylacétyle en position 5 du résidu sucre, ou

B. la réaction d'un composé de formule III :

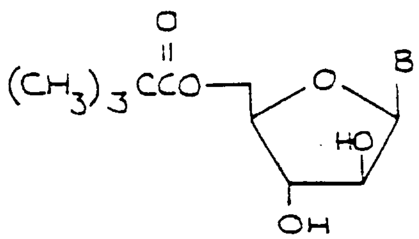

(III)

où B est une base purique ou pyrimidinique (autre que 5-prop-1-ynyluracile) avec du 5-prop-1-ynyluracile et, facultativement, après ou simultanément, quand le composé résultant est un composé de formule I, conversion en un sel de celui-ci ou, quand le composé résultant est un sel, conversion en un sel différent ou en un composé de formule I, et ensuite, mise en association du composé résultant avec un excipient pharmaceutiquement acceptable de celui-ci.